# EUROPÄISCHE PATENTSCHRIFT

(11) **EP 1 104 262 B1**
(45) Veröffentlichungstag und Bekanntmachung des Hinweises auf die Patenterteilung: **27.10.2004**
(21) Anmeldenummer: 99941521.9
(22) Anmeldetag: 05.08.1999
(51) Int. Cl.: A61B 18/14

(54) **ELEKTROCHIRURGISCHES ROTIERENDES SCHNEIDGERÄT**
ELECTROSURGICAL ROTATING CUTTING INSTRUMENT
BISTOURI ELECTROCHIRURGICAL ROTATIF

(30) Priorität: 10.08.1998 DE 19836074
(43) Veröffentlichungstag der Anmeldung: 06.06.2001
(73) Patentinhaber: Karl Storz GmbH & Co. KG, 78532 Tuttlingen (DE)
(72) Erfinder: DITTRICH, Horst, D-78194 Immendingen (DE); DOLL, Frank, D-78589 Dürbheim (DE); GMINDER, Frank, D-78647 Trossingen (DE)
(74) Vertreter: Lindner, Michael, Dipl.-Ing.
(86) Internationale Anmeldenummer: PCT/EP1999/005669
(87) Internationale Veröffentlichungsnummer: WO 2000/009026

(56) Entgegenhaltungen:
- WO-A-96/24296
- US-A- 5 693 051

## Beschreibung

Die Erfindung betrifft ein medizinisches Instrument, das an seinem distalen Ende ein mit Hochfrequenz(HF)-Energie beaufschlagbares Werkzeug, einen HF-Stromanschluß und eine HF-Stromzuführung aufweist, die sich von einem proximalen Endbereich des Instruments zu dessen distalen Ende erstreckt, wobei die HF-Stromzuführung als Antriebswelle zum rotatorischen Antrieb des Werkzeugs ausgebildet ist und eine Kontakteinrichtung vorgesehen ist, die mit dem HF-Stromanschluß elektrisch verbunden ist und die Antriebswelle elektrisch kontaktiert.

Derartige medizinische Instrumente sind bspw. aus WO 96/24296 bekannt. Sie werden üblicherweise zum Abtragen von Weichgewebe eingesetzt, wobei das Instrument über ein rotierendes oder feststehendes Schneidwerkzeug verfügt, das mit HF-Energie, d.h. mit einer Hochfrequenz(HF)-Spannung bzw. einem HF-Strom beaufschlagt ist. Der bei Kontakt mit dem Gewebe fließende HF-Strom sorgt für eine starke Erhitzung im Nah-Bereich um das Schneidwerkzeug (Elektrode), so daß das Gewebe abgetragen und an der Schnittstelle koaguliert wird. Damit werden gleichzeitig mit der Gewebeabtragung Blutgefäße geschlossen, so daß größere Blutungen vermieden werden.

Die Übertragung der HF-Spannung auf die rotierende Antriebswelle erfolgt in der in WO 96/24296 offenbarten Ausführungsform über Bürstenkontakte. Bei dieser Art der Übertragung besteht jedoch die Gefahr von Funkenbildung, die gerade bei medizinischen Instrumenten nachteilig ist.

Vor diesem Hintergrund liegt der Erfindung die Aufgabe zugrunde, eine medizinische Vorrichtung der eingangs genannten Art so weiterzubilden, daß die Übertragung von HF-Energie auf die Antriebswelle verbessert wird.

Die der Erfindung zugrundeliegende Aufgabe wird von einem medizinischen Instrument der eingangs genannten Art gelöst, bei dem die Kontakteinrichtung ein Federmittel umfaßt, und das Federmittel als U-förmiges Element ausgebildet ist, dessen beiden Schenkel jeweils ein Kontaktelement tragen und dieses in einer in Richtung der Antriebswelle wirkenden Kraft beaufschlagen.

Diese Maßnahme hat den Vorteil, daß eine sehr gute Kontaktierung zwischen den Kontaktelementen und der Antriebswelle hergestellt wird, so daß eine Funkenbildung vermieden werden kann. Darüber hinaus reduziert sich aufgrund der guten Kontaktfläche die Entstehung von Wärme, was ebenfalls nicht nur im Hinblick auf die Verschleißfestigkeit Vorteile bietet.

Die der Erfindung zugrundeliegende Aufgabe wird auch von einem medizinischen Instrument der eingangs genannten Art gelöst, bei dem die Kontakteinrichtung ein Federmittel umfaßt, das da Kontaktelement mit einer in Richtung der Antriebswelle wirkenden Kraft beaufschlagt, das Kontaktelement in einer Kontaktelementführung geführt ist, das Federmittel so angeordnet ist, daß die Kraft auch in Richtung einer Führungsfläche der Kontaktelementführung wirkt und die Führungsfläche als elektrische Verbindungsfläche zwischen Kontaktelementführung und Kontaktelement ausgebildet ist, so daß sich eine elektrische Verbindung zwischen dem HF-Stromanschluß und dem Kontaktelement über die Kontaktelementführung ergibt.

Auch diese alternative Ausführungsform hat die oben genannten Vorteile einer sehr guten Kontaktierung der Antriebswelle.

Die der Erfindung zugrundeliegende Aufgabe wird ferner von einem medizinischen Instrument der eingangs genannten Art gelöst, bei dem die Kontakteinrichtung ein Gehäuse umfaßt, das einen Längsabschnitt der Antriebswelle aufnimmt und in dem ein elektrisch leitfähiges Fluid fluiddicht enthalten ist, so daß eine elektrische Verbindung zwischen der Antriebswelle, dem Fluid und folglich dem HF-Stromanschluß bewirkt wird.

Diese alternative Ausführungsform hat den Vorteil, daß die durch Reibung des Kontaktelements an der schnell drehenden Antriebswelle entstehende Wärme deutlich verringert wird. Da das elektrisch leitfähige Fluid die Antriebswelle in Umfangsrichtung vollständig umschließt, ist die erreichte Größe der Kontaktfläche maximal. Ferner hat diese Ausführungsform den Vorteil, daß zwischen Kontaktelement und Antriebswelle kein Luftspalt vorhanden ist.

In einer bevorzugten Weiterbildung der Erfindung ist die elektrische Kontaktierung mit der Antriebswelle als flächige Kontaktierung ausgebildet.

Dies hat den Vorteil, daß die elektrische Feldstärke im Bereich dieser Verbindung trotz der hohen HF-Spannung gering ist und die Entstehung von Funkenentladungen verhindert wird. Insbesondere wird durch ein möglichst großflächiges Anliegen (Kontakt) und einen kleinen Luftspalt die Stromübertragung optimiert. Darüber hinaus entstehen durch Funkenentladungen hochfrequente elektromagnetische Wellen, die zu Störungen in anderen elektrischen Geräten führen können. Gerade hochsensible medizinische Geräte könnten durch derartige Funkenentladungen empfindlich gestört werden.

In einer bevorzugten Weiterbildung ist die elektrische Kontaktierung als flächige Kontaktierung über zumindest einen Teilumfangsbereich der Antriebswelle ausgebildet.

Dies hat den Vorteil, daß sich eine sehr große Verbindungsfläche realisieren läßt, so daß das elektrische Feld sehr klein ist, wobei sich die Verbindungsfläche selbst im wesentlichen beliebig durch Ausdehnung in Längsrichtung der Antriebswelle vergrößern läßt. Ein weiterer Vorteil ist darin zu sehen, daß die Reibungskräfte pro Flächeneinheit verringert werden, so daß einerseits die Abnutzung der Kontaktierungsfläche verringert wird und andererseits die durch Reibung entstehende Wärme vermindert wird. Ferner wird dadurch auch die durch Stromübertragung erzeugte Wärme deutlich vermindert.

In einer bevorzugten Weiterbildung umfaßt die Kontakteinrichtung ein verlagerbares Kontaktelement, das in einer Kontaktelementführung geführt ist und mit einer Kontaktfläche flächig an der Antriebswelle anliegt, wobei bevorzugt die Kontaktfläche des Kontaktelements an die Form der Antriebswelle angepaßt ist.

Dies hat den Vorteil, daß in konstruktiv einfacher Weise eine Kontaktierung der Antriebswelle mit einer großen Kontaktfläche erzielbar ist. Je nach Ausführungsform kann sich die Kontaktfläche des Kontaktelements entlang einer Hälfte des Umfangs der Antriebswelle erstrecken. Darüber hinaus läßt sich mit der Kontaktelementführung die durch Reibung und die Stromübertragung entstehende Wärme gut ableiten.

Bevorzugt ist das Federmittel so angeordnet, daß die Kraft auch in Richtung einer Führungsfläche der Kontaktelementführung wirkt, und daß die Führungsfläche als elektrische Kontaktierungsfläche zwischen Kontaktelementführung und Kontaktelement ausgebildet ist.

Dies hat den Vorteil, daß das Federmittel das Kontaktelement sicher gegen die Führungsfläche der Kontaktelementführung gedrückt wird, so daß auch zwischen diesen beiden Teilen eine gute elektrische und thermische Verbindung sichergestellt wird. Der HF-Strom läßt sich somit über die Führungsfläche und dem daran anliegenden Kontaktelement der Antriebswelle zuführen.

In einer bevorzugten Weiterbildung enthält das Kontaktelement ein Metall, vorzugsweise Kupfer, und ist als Kontaktblock gefertigt.

Dies hat den Vorteil, daß das Kontaktelement eine sehr gute elektrische Leitfähigkeit aufweist, wobei jedoch eine hohe Verschleißfestigkeit erhalten bleibt.

In einer bevorzugten Weiterbildung ist die Führungsfläche der Kontaktelementführung vergoldet. Bevorzugt ist der mit der Kontakteinrichtung zusammenwirkende Umfangsbereich der Antriebswelle mit einem elektrisch gut leitfähigen, widerstandsfähigen und korrosionsbeständigen Material, vorzugsweise Gold, beschichtet.

Dies hat den Vorteil, daß die elektrische Verbindung zwischen Kontakteinrichtung und Antriebswelle weiter verbessert wird, so daß auch die Gefahr der Funkenentladung weiter verringert wird.

Weitere Vorteile und Ausgestaltungen der Erfindung ergeben sich aus der Beschreibung und der beiliegenden Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nun anhand von Ausführungsbeispielen mit Bezug auf die Zeichnungen näher erläutert. Dabei zeigen:
- Fig. 1: eine schematische Seitenansicht eines medizinischen Instruments;
- Fig. 2: ein Ausschnitt des in Fig. 1 gezeigten medizinischen Instruments im Bereich einer Kontakteinrichtung in perspektivischer Ansicht;
- Fig. 3: eine schematische Schnittdarstellung der Kontakteinrichtung;
- Fig.4: eine perspektivische schematische Darstellung einer Kontakteinrichtung gemäß einem zweiten Ausführungsbeispiels; und
- Fig. 5: eine schematische Schnittdarstellung einer Kontakteinrichtung gemäß einem weiteren Ausführungsbeispiel.

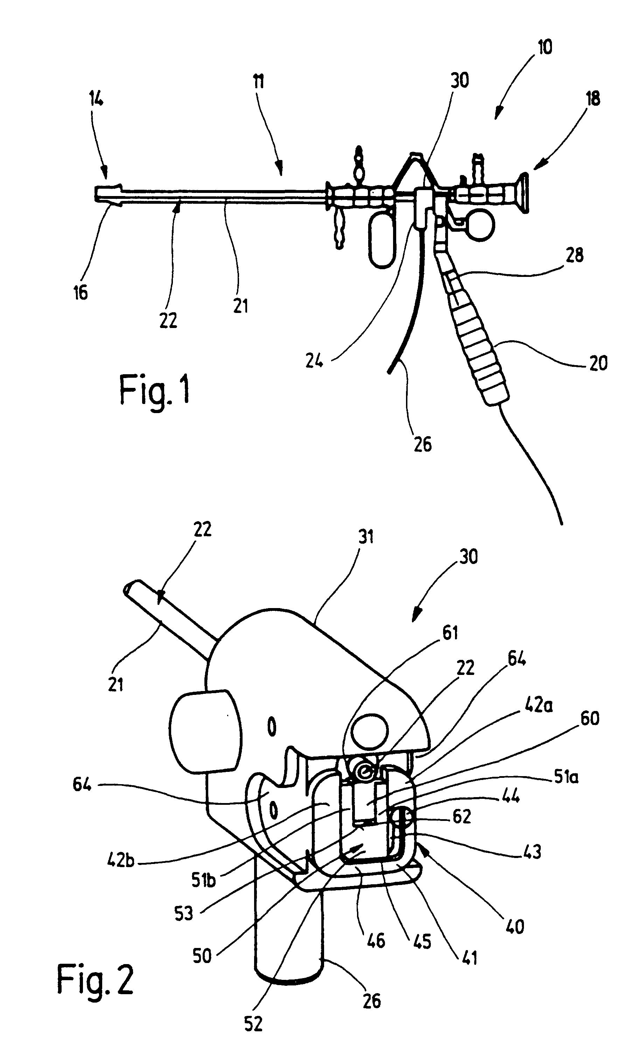

In Fig. 1 ist ein lediglich schematisch angedeutetes medizinisches Instrument mit dem Bezugszeichen 10 gekennzeichnet. Dieses medizinische Instrument wird bspw. zur Gewebeentnahme an der Prostata verwendet und ist im vorliegenden Ausführungsbeispiel als endoskopisches Instrument 11 ausgebildet. Der grundsätzliche Aufbau eines solchen Instruments 11 ist bekannt, so daß darauf nicht näher eingegangen werden soll.

Das Instrument 11 weist an seinem distalen Ende 14 ein ebenfalls nur schematisch angedeutetes Werkzeug 16 auf, das im vorliegenden Ausführungsbeispiel als aufsteckbares - und auswechselbares -, bspw. kegelförmiges Schneidwerkzeug mit integrierten Elektroden ausgebildet ist. Im Zusammenhang mit der vorliegenden Erfindung ist unter dem Begriff Schneidwerkzeug ein Werkzeug zu verstehen, das allgemein zum Abtragen und Koagulieren von Gewebe bspw. durch Schneiden oder Fräsen geeignet ist. Das Instrument 11 weist im Bereich seines proximalen Endes 18 einen Elektromotor 20 auf, der über eine zumindest teilweise dargestellte Antriebswelle 22 und eine Abtriebswelle 28 das drehbar gelagerte Schneidwerkzeug 16 mit einer bevorzugten Drehzahl von ca. 1000 Umdrehungen pro Minute antreibt. Des weiteren ist im Bereich des proximalen Endes 18 des Instruments 11 ein elektrischer Anschluß 24 vorgesehen, an den eine elektrische Leitung 26 anschließbar ist. Diese elektrische Leitung 26 ist ihrerseits mit einem nicht dargestellten Hochfrequenz(HF)-Generator verbunden. Sowohl der elektrische Anschluß 24 als auch eine nicht dargestellte Kopplungseinrichtung zur Kopplung der Antriebswelle 22 und der Abtriebswelle 28 des Motors 20 sind Teil eines Anschlußstücks, das in Fig. 1 mit dem Bezugszeichen 30 gekennzeichnet ist und in Fig. 2 in vergrößerter Darstellung gezeigt ist.

Das in Fig. 2 teilweise dargestellte Anschlußstück 30 umfaßt ein Gehäuse 31, in dem die Antriebswelle 22 endet. Rechtwinklig zur Antriebswelle 22 mündet die elektrische Leitung 26 in das Gehäuse 31.

Im Gehäuse 31 ist eine Kontakteinrichtung 40 vorgesehen, die einen U-förmigen, elektrisch isolierenden Träger 41 umfaßt. Dieser Träger 41 ist fest mit dem Gehäuse 31 verbunden und so angeordnet, daß das offene Ende der U-Form der Antriebswelle 22 zugewandt ist. Einer von zwei Schenkeln 42a, 42b des Trägers 41 ist mit einer Ausnehmung 43 versehen, in die eine elektrische Leitung 44 hineinragt. Die Leitung 44 ist einerseits mit der Leitung 26 und andererseits mit einem Kontaktierungselement 45 verbunden, das sich ausgehend von der Leitung 44 entlang des einen Schenkels 42a und einer Grundfläche 46 des Trägers 41 bis hin zum anderen Schenkel 42b erstreckt.

Der U-förmige Träger 41 nimmt zwischen seinen Schenkeln 42a, 42b ein Führungselement 50 auf, das an beiden Schenkeln 42a, 42b und im Bereich der Grundfläche 46 an dem Kontaktierungselement 45 anliegt. Das Führungselement 50 ist entweder vollständig aus einem elektrisch leitfähigen Material, wie bspw. Kupfer gefertigt oder weist lediglich eine elektrisch leitfähige Schicht, bspw. aus Gold, auf einem beliebigen, vorzugsweise thermisch gut leitenden Grundkörper, bspw. aus Messing, auf. Das Führungselement 50 weist ebenfalls eine U-Form auf, wobei jeweils ein Schenkel 51a, 51b an dem entsprechenden Schenkel 42a bzw. 42b des Trägers 41 anliegt. Eine Basis 52 des Führungselements 50 ist der Grundfläche 46 des Trägers 41 zugewandt und liegt auf dem Kontaktierungselement 45 auf, das seinerseits an der Grundfläche 46 anliegt.

In einem seitlich durch die beiden Schenkel 51a, 51b begrenzten Raum 53 ist ein blockförmiges Kontaktelement 60 verlagerbar aufgenommen, wobei eine der Antriebswelle 22 zugewandte Kontaktfläche 61 des Kontaktelements 60 mit der Antriebswelle 22 in elektrischem Kontakt steht. Um eine sichere Kontaktierung zu gewährleisten, ist das Kontaktelement 60 mit einer Kraft beaufschlagt, die in Richtung der Antriebswelle 22 wirkt. Diese Kraft wird vorzugsweise von einer Feder erbracht, die sich einerseits an der Basis 52 des Führungselements 50 und andererseits an der der Basis zugewandten Seite des Kontaktelements 60 abstützt. Die Feder ist in Fig. 2 lediglich als Punkt schematisch dargestellt und mit dem Bezugszeichen 62 gekennzeichnet.

Die Kontakteinrichtung 40 dient insgesamt dazu, den über die Leitung 26 zugeführten HF-Strom über die Leitung 44, das Kontaktierungselement 45, das leitende Führungselement 50 und das Kontaktelement 60 der Antriebswelle 22 zuzuführen. Da die Antriebswelle 22 aus einem leitfähigen Material gefertigt ist bzw. zumindest eine elektrisch leitfähige Schicht aufweist und damit als Spannungs- bzw. Stromzuführung 21 dient, gelangt der HF-Strom dann zu den in dem Werkzeug 16 integrierten Elektroden.

Fig. 2 läßt seitlich am Gehäuse 31 vorgesehene Ausnehmungen 64 erkennen, die zur Ankopplung eines Gehäusedeckels dienen. Das Gehäuse 31 umfaßt bspw. ein Getriebe, das eine Verbindung zwischen der Abtriebswelle 28 und der Antriebswelle 22 ermöglicht.

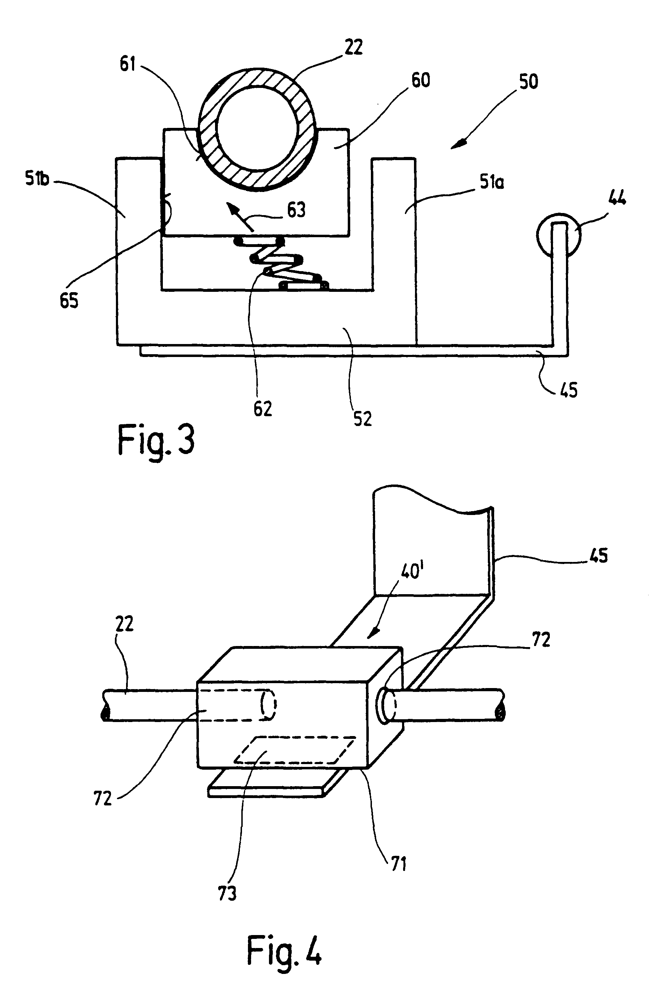

Mit Bezug auf Fig. 3 wird nun das Führungselement 50 näher erläutert, wobei gleiche Teile wie in Fig. 2 mit gleichen Bezugszeichen gekennzeichnet sind.

Wie bereits erwähnt, liegt die Kontaktfläche 61 des Kontaktelements 60 an der Antriebswelle 22 an. Um eine möglichst große flächige Verbindung zu erzielen, ist die Kontaktfläche 61 an die äußere Form der Antriebswelle 22 angepaßt. Im vorliegenden Ausführungsbeispiel erstreckt sich die Kontaktfläche 61 quer zur Längsrichtung der Antriebswelle 22 bevorzugt über eine Hälfte des Umfangs und weist damit eine halbzylindrische Form auf. Selbstverständlich ist es auch denkbar, die Kontaktfläche 61 so auszubilden, daß sie sich über einen kleineren Teil des Umfangs erstreckt.

Das Kontaktelement 60 wird durch die beiden Schenkel 51a, 51b des Führungselements 50 geführt und gegen ein Verkippen bzw. Verdrehen um die Längsachse der Antriebswelle 22 gesichert.

Das Kontaktelement 60 wird wie bereits erwähnt mit einer Federkraft beaufschlagt, die durch einen Pfeil 63 gekennzeichnet ist. Diese Federkraft wird von der schematisch angedeuteten Feder 62 erbracht, die zwischen der Basis 52 und dem Kontaktelement 60 liegt. Die schräg zum Radius der Antriebswelle 22 wirkende Federkraft 63 drückt das Kontaktelement 60 nicht nur mit dessen Kontaktfläche 61 an die Antriebswelle 22, sondern auch mit einer seitlichen Kontaktfläche 65 an den Schenkel 51b des Führungselements 50, so daß eine flächige Verbindung geschaffen wird. Um die Kontaktierung zwischen dem Kontaktelement 60 und dem Schenkel 51b zu verbessern und vor Korrosion zu schützen, wird bevorzugt die Kontaktfläche 65 mit einer besonders gut leitenden Schicht, bspw. Gold, beschichtet. Selbstverständlich läßt sich auch die Kontaktfläche 61 mit einer gut leitenden und möglichst verschleißfesten Schicht beschichten. Damit ergibt sich ein Stromfluß über die Leitung 44 und das Kontaktierungselement 45 zum Führungselement 50 und von diesem über dessen Schenkel 51b über die Kontaktierungsfläche 65 und das Kontaktelement 60 zur Kontaktfläche 61 und dann zur Antriebswelle 22. Die rohrförmig aufgebaute Antriebswelle 22 führt dann den Strom zu dem Werkzeug 16. Gleichzeitig sorgt die Antriebswelle 22 dafür, daß das Werkzeug 16 rotiert. Neben der Kontaktfläche 65 zwischen dem Kontaktelement 60 und dem Führungselement 50 läßt sich die Feder 62 entweder alternativ oder zusätzlich als Stromzuführung ausbilden. Hierfür ist die Feder 62 aus einem elektrisch leitfähigen Material herzustellen bzw. zumindest zu beschichten, wobei die Federauflageflächen sowohl am Kontaktelement 60 als auch am Führungselement 50 möglichst mit einem gut leitfähigen Material zu beschichten sind.

Es versteht sich, daß die Feder 62 auch durch ein eine Vielzahl von einzelnen Federn umfassendes Federpaket ersetzt werden kann. Darüber hinaus ist es denkbar, die schräg zum Radius wirkende Federkraft 63 durch zwei einzelne Federn bzw. Federpakete zu erzielen, wobei eine Feder in radialer Richtung und die andere Feder senkrecht zur Kontaktfläche 65 wirkt.

Eine weitere Ausführungsform der Erfindung ist in Fig. 4 dargestellt. Deutlich zu erkennen ist eine Kontakteinrichtung 40', die ein quaderförmiges Gehäuse 71 aufweist. Dieses Gehäuse 71 wird von der Antriebswelle 22 gemäß einer ersten Variante vollständig durchgriffen, wobei die hierfür benötigten beiden Öffnungen 72 Wellendichtungen umfassen, um das Gehäuse 71 fluiddicht auszugestalten. Das Gehäuse 71 ist mit einem Fluid, vorzugsweise einem flüssigen Metall, bspw. Quecksilber, gefüllt, das eine gute elektrische Leitfähigkeit besitzt. Durch entsprechende Wahl der Fluidmenge ist der innerhalb des Gehäuses 71 verlaufende Längsabschnitt der Antriebswelle 22 vollständig von dem Fluid umgeben. Gemäß einer bevorzugten zweiten Variante endet die Antriebswelle 22, wie gestrichelt dargestellt, innerhalb des Gehäuses 71, so daß lediglich auf einer Gehäuseseite eine Öffnung 72 mit einer Wellendichtung vorzusehen ist. Das Gehäuse 71 weist bei beiden Varianten eine Kontaktfläche 73 auf, die eine elektrische Verbindung zwischen dem Kontaktierungselement 45 und dem innerhalb des Gehäuses 71 befindlichen Fluid herstellt.

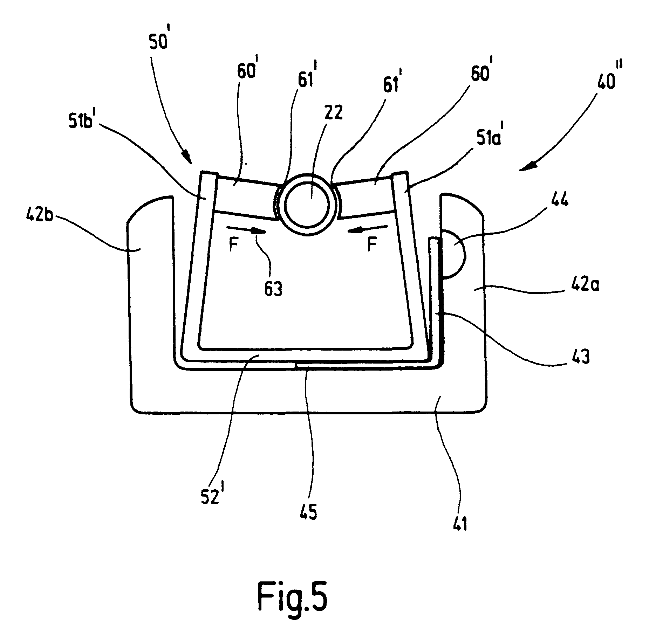

Eine weitere Ausführungsform der Erfindung ist in Fig. 5 schematisch dargestellt. Deutlich zu erkennen ist eine Kontakteinrichtung 40", die in dem in Fig. 2 gezeigten Anschlußstück verwendbar ist und im wesentlichen mit der in Fig. 3 gezeigten und ausführlich beschriebenen Kontakteinrichtung 40 übereinstimmt. Aus diesem Grund wird nachfolgend nur auf die Unterschiede eingegangen.

In der Ausführungsform gemäß Fig. 5 ist ein Führungselement 50' vorgesehen, das eine U-Form besitzt, so daß sich zwei Schenkel 51a' und 51b' und eine Basis 52' ausbilden. An den der Basis abgewandten Enden beider Schenkel 51a', 51b' ist jeweils ein Kontaktelement 60' angebracht. Beide Kontaktelemente 60' erstrecken sich zu der Antriebswelle 22 und stehen über Kontaktflächen 61' mit der Antriebswelle 22 in elektrischem Kontakt. Um eine sichere Kontaktierung zu gewährleisten, sind beide Kontaktelemente 60' mit einer Kraft 63 beaufschlagt, die in Richtung der Antriebswelle 22 wirkt. In der Fig. 5 ist diese Kraft mit einem Pfeil F gekennzeichnet.

Bei der vorliegenden Ausführungsform wird die Kraft F von den beiden Schenkeln 51a', 51b' des Führungselements 50' erbracht.

Das Führungselement 50' übernimmt damit zusätzlich die Aufgabe der Feder 62, die in der in Fig. 3 gezeigten Ausführungsform verwendet wird. Um diese Federfunktion zu erbringen, sollte das Führungselement 50' aus einem federnden bzw. elastischen Material, das zudem elektrisch leitfähig ist, gefertigt sein.

Der über die Leitung 26 zugeführte HF-Strom wird - wie bei den mit Bezug auf die Fig. 2 und 3 beschriebenen Ausführungsformen - über die Leitung 44, das Kontaktierungselement 45, das leitende Führungselement 50' und die Kontaktelemente 60' der Antriebswelle 22 zugeführt.

Selbstverständlich sind auch andere Ausführungsformen denkbar, die eine Kontaktierung vorzugsweise einer größeren Fläche einer Antriebswelle ermöglichen. Die großflächige Verbindung hat dabei den Vorteil, daß durch große elektrische Feldstärken verursachte Funkenentladungen unterbunden werden. Damit wird gleichzeitig einer Zerstörung der Antriebswelle 22 aufgrund solcher Funkenentladungen entgegengewirkt.

Die beschriebenen Ausführungsformen sind für monopolare Anwendungen ausgestaltet. Selbstverständlich läßt sich die Erfindung auch bei medizinischen Instrumenten für bipolare Anwendungen einsetzen. In diesem Fall können beipielsweise zwei erfindungsgemäße Kontakteinrichtungen 40 eingesetzt werden, wobei die beiden HF-Spannungszuführungen beispielsweise als zwei koaxial zueinander angeordnete Wellen ausgebildet sind.

## Patentansprüche

1. Medizinisches Instrument, das an seinem distalen Ende (14) ein mit einer Hochfrequenz (HF)-Energie beaufschlagbares Werkzeug (16), sowie einen HF-Stromanschluß (24) und eine HF-Stromzuführung (21) aufweist, die sich von einem proximalen Endbereich (18) des Instruments zu dessen distalen Ende (14) erstreckt, wobei die HF-Stromzuführung (21) als Antriebswelle (22) zum rotatorischen Antrieb des Werkzeugs (16) ausgebildet ist und eine Kontakteinrichtung (40'') vorgesehen ist, die mit dem HF-Stromanschluß (24) elektrisch verbunden ist und die Antriebswelle (22) elektrische kontaktiert, **dadurch gekennzeichnet, daß** die Kontakteinrichtung (40'') ein Federmittel (50') umfaßt, und daß das Federmittel (50') als U-förmiges Element ausgebildet ist, dessen beiden Schenkel (51a', 51b') jeweils ein Kontaktelement (60') tragen und dieses mit einer in Richtung der Antriebswelle (22) wirkenden Kraft beaufschlagen.

2. Medizinisches Instrument, das an seinem distalen Ende (14) ein mit einer Hochfrequenz (HF)-Energie beaufschlagbares Werkzeug (16), sowie einen HF-Stromanschluß (24) und eine HF-Stromzuführung (21) aufweist, die sich von einem proximalen Endbereich (18) des Instruments zu dessen distalen Ende (14) erstreckt, wobei die HF-Stromzuführung (21) als Antriebswelle (22) zum rotatorischen Antrieb des Werkzeugs (16) ausgebildet ist, und eine Kontakteinrichtung (40) vorgesehen ist, die mit dem HF-Stromanschluß (24) elektrisch verbunden ist und die Antriebswelle (22) elektrische kontaktiert, **dadurch gekennzeichnet, daß** die Kontakteinrichtung (40) ein Federmittel (62) umfaßt, das das Kontaktelement (60) mit einer in Richtung der Antriebswelle (22) wirkenden Kraft (63) beaufschlagt, daß das Kontaktelement (60) in einer Kontaktelementführung (50) geführt ist, daß das Federmittel (62) so angeordnet ist, daß die Kraft auch in Richtung einer Führungsfläche (65) der Kontaktelementführung (50) wirkt, und daß die Führungsfläche (65) als elektrische Verbindungsfläche zwischen Kontaktelementführung (50) und Kontaktelement (60) ausgebildet ist, so daß sich eine elektrische Verbindung zwischen dem HF-Stromanschluß (24) und dem Kontaktelement (60) über die Kontaktelementführung (50) ergibt.

3. Medizinisches Instrument nach Anspruch 1 oder 2, **dadurch gekennzeichnet, daß** die elektrische Kontaktierung als flächige Kontaktierung ausgebildet ist.

4. Medizinisches Instrument nach Anspruch 1, 2 oder 3, **dadurch gekennzeichnet, daß** die elektrische Kontaktierung als flächige Kontaktierung über zumindest einen Teilumfangsbereich der Antriebswelle (22) ausgebildet ist.

5. Medizinisches Instrument nach einem der vorhergehenden Ansprüche, **dadurch gekennzeichnet, daß** das Kontaktelement (60; 60') mit einer Kontaktfläche (61) flächig an der Antriebswelle (22) anliegt.

6. Medizinisches Instrument nach Anspruch 5, **dadurch gekennzeichnet, daß** die Kontaktfläche (61; 61') des Kontaktelements (60; 60') an die Form der Antriebswelle (22) angepaßt ist.

7. Medizinisches Instrument nach Anspruch 1, **dadurch gekennzeichnet, daß** das Federmittel (50') aus einem elektrisch leitfähigen Material besteht und eine elektrische Verbindung zwischen dem HF-Stromanschluß und dem Kontaktelement (60'') ermöglicht.

8. Medizinisches Instrument Anspruch 1 oder 2, **dadurch gekennzeichnet, daß** das Kontaktelement (60; 60') ein elektrisch gut leitfähiges Material, vorzugsweise Kupfer, enthält und als Kontaktblock gefertigt ist.

9. Medizinisches Instrument nach Anspruch 2, **dadurch gekennzeichnet, daß** zumindest die Führungsfläche (65) der Kontaktelementführung (50) vergoldet ist.

10. Medizinisches Instrument nach einem der vorhergehenden Ansprüche, **dadurch gekennzeichnet, daß** der mit der Kontakteinrichtung zusammenwirkende Umfangsbereich der Antriebswelle (22) mit einem elektrisch gut leitfähigen und widerstandsfähigen Material, vorzugsweise Gold, beschichtet ist.

11. Medizinisches Instrument, das an seinem distalen Ende (14) ein mit einer Hochfrequenz (HF)-Energie beaufschlagbares Werkzeug (16), sowie einen HF-Stromanschluß (24) und eine HF-Stromzuführung (21) aufweist, die sich von einem proximalen Endbereich (18) des Instruments zu dessen distalen Ende (14) erstreckt, wobei die HF-Stromzuführung (21) als Antriebswelle (22) zum rotatorischen Antrieb des Werkzeugs (16) ausgebildet ist, und eine Kontakteinrichtung (40') vorgesehen ist, die mit dem HF-Stromanschluß (24) elektrisch verbunden ist und die Antriebswelle (22) elektrisch kontaktiert, **dadurch gekennzeichnet, daß** die Kontakteinrichtung (40') ein Gehäuse (71) umfaßt, das einen Längsabschnitt der Antriebswelle (22) aufnimmt und in dem ein elektrisch leitfähiges Fluid fluiddicht enthalten ist, so daß eine elektrische Verbindung zwischen der Antriebswelle (22), dem Fluid und folglich dem HF-Stromanschluß (24) bewirkt wird.

## Claims

1. Medical instrument comprising a tool (16) at its distal end (14) to be supplied with high frequency (HF) power, a HF power terminal (24) and a HF power supply line (21) extending from a proximal end region (18) of the instrument to its distal end (14), wherein the HF power supply line (21) is configured as a drive shaft (22) for rotary drive of the tool (16) and wherein contact means (40'') are provided which are electrically connected to the HF power terminal (24) and electrically contact the drive shaft (22), **characterized in that** the contact means comprise a spring means (50') configured as a U-shaped element, whose two sidepieces (51a', 51b') carry a contact element (60') and urge it with a force acting in the direction of the drive shaft (22).

2. Medical instrument comprising a tool (16) at its distal end (14) to be supplied with high frequency (HF) power, a HF power terminal (24) and a HF power supply line (21) extending from a proximal end region (18) of the instrument to its distal end (14), wherein the HF power supply line (21) is configured as a drive shaft (22) for rotary drive of the tool (16) and wherein contact means (40) are provided which are electrically connected to the HF power terminal (24) and electrically contact the drive shaft (22), **characterized in that** the contact means (40) comprise spring means (62) which urge the contact element (60) with a force acting in the direction of the drive shaft (22), the contact element (60) is guided in a contact element guide (50), the spring means (62) are arranged such that a force also acts in the direction of a guide surface (65) of the contact element guide (50) and the guide surface (65) is configured as an electrical contact surface between the contact element guide (50) and the contact element (60), so that an electrical connection results between the HF power terminal (24) and the contact element (60) via the contact element guide (50).

3. Medical instrument of claim 1 or 2, **characterized in that** the electrical contacting is configured as surface contact.

4. Medical instrument of claim 1, 2 or 3, **characterized in that** the electrical contacting is configured as a surface contact over at least a partial circumference of the drive shaft (22).

5. Medical instrument of any of the preceding claims, **characterized in that** the contact element (60, 60') engages with a contact surface (61) over a surface of the drive shaft (22).

6. Medical instrument of claim 5, **characterized in that** the contact surface (61, 61') of the contact element (60; 60') is adapted to the shape of the drive shaft (22).

7. Medical instrument of claim 1, **characterized in that** the spring means (50') consist of an electrically conductive material and an electrical connection is made possible between the HF power terminal and the contact element (60'').

8. Medical instrument of claim 1 or 2, **characterized in that** the contact element (60; 60') contains a material of good electrical conductivity, preferably copper and is produced as a contact block.

9. Medical instrument of claim 2, **characterized in that** at least the guide surface of the contact element guide is gold-plated.

10. Medical instrument of any of the preceding claims, wherein the circumferential region the drive shaft (22) engaging with the contact means is coated with a resistant material having a good electrical conductivity, preferably gold.

11. Medical instrument comprising a tool (16) at its distal end (14) to be supplied with high frequency (HF) power, a HF power terminal (24) and a HF power supply line (21) extending from a proximal end region (18) of the instrument to its distal end (14), wherein the HF power supply line (21) is configured as a drive shaft (22) for rotary drive of the tool (16) and wherein contact means (40') are provided which are electrically connected to the HF power terminal (24) and electrically contact the drive shaft (22), **characterized in that** the contact means (40') comprise a housing (71) which receives a section of the drive shaft (22) and in which an electrically conductive fluid is contained in fluid-tight manner, so that an electrical connection between the drive shaft (22), the fluid and consequently the HF power terminal (24) results.

## Revendications

1. Instrument médical, qui comprend à son extrémité distale (14) un instrument (16) alimenté en énergie à haute fréquence (HF), ainsi qu'un raccordement électrique HF (24) et une amenée de courant HF (21), qui s'étend d'une extrémité proximale (18) de l'instrument jusqu'à son extrémité distale (14), sachant que l'amenée de courant HF (21) est conçue comme un arbre d'entraînement (22) pour un mécanisme d'entraînement rotatif de l'instrument (16) et un dispositif de contact (40") est prévu qui est relié électriquement au raccordement électrique HF (24) et vient au contact de l'arbre d'entraînement (22) de manière électrique, **caractérisé en ce que** le dispositif de contact (40") comprend un ressort (50') et **en ce que** le ressort (50') est conçu comme un élément en forme de U dont les deux branches (51a', 51b') portent respectivement un élément de contact (60') et alimentent celui-ci avec une force agissant en direction de l'arbre d'entraînement (22).

2. Instrument médical, qui comprend à son extrémité distale (14) un instrument (16) alimenté en énergie à haute fréquence (HF), ainsi qu'un raccordement électrique HF (24) et une amenée de courant HF (21), qui s'étend d'une extrémité proximale (18) de l'instrument jusqu'à son extrémité distale (14), sachant que l'amenée de courant HF (21) est conçue comme un arbre d'entraînement (22) pour un mécanisme d'entraînement rotatif de l'instrument (16) et un dispositif de contact (40") est prévu qui est relié électriquement au raccordement électrique HF (24) et vient au contact de l'arbre d'entraînement (22) de manière électrique, **caractérisé en ce que** le dispositif de contact (40) comprend un ressort (62) qui alimente l'élément de contact (60) avec une force (63) agissant en direction de l'arbre d'entraînement (22), **en ce que** l'élément de contact (60) est guidé dans un conduit d'élément de contact (50), **en ce que** le ressort (62) est agencé de telle sorte que la force agit également en direction d'une surface conductrice (65) du conduit d'élément de contact (50), et **en ce que** la surface conductrice (65) est conçue en tant que surface de connexion électrique entre le conduit d'élément de contact (50) et l'élément de contact (60) de telle sorte qu'une connexion électrique entre le raccordement électrique HF (24) et l'élément de contact (60) se produit via le conduit d'élément de contact (50).

3. Instrument médical selon la revendication 1 ou 2, caractérisé en ce l'établissement de contact électrique est formé en tant qu'établissement de contact en nappe.

4. Instrument médical selon la revendication 1, 2 ou 3, **caractérisé en ce que** l'établissement de contact électrique est conçu en tant qu'établissement de contact en nappe via au moins une sous-partie du pourtour de l'arbre d'entraînement (22).

5. Instrument médical selon l'une des revendications précédentes, **caractérisé en ce que** l'élément de contact (60; 60') est ajusté avec une surface conductrice (61) en nappe à l'arbre d'entraînement (22).

6. Instrument médical selon la revendication 5, **caractérisé en ce que** la surface conductrice (61; 61') de l'élément de contact (60; 60') est adaptée à la forme de l'arbre d'entraînement (22).

7. Instrument médical selon la revendication 1, **caractérisé en ce que** le ressort (50') se compose d'un matériau conducteur électrique et permet une connexion électrique entre le raccordement électrique HF et l'élément de contact (60').

8. Instrument médical selon la revendication 1 ou 2, **caractérisé en ce que** l'élément de contact (60; 60') comprend un matériau bon conducteur électrique, de préférence du cuivre, et fabriqué en tant que bloc de contact.

9. Instrument médical selon la revendication 2, **caractérisé en ce qu'**au moins la surface conductrice (65) du conduit d'élément de contact (50) est dorée.

10. Instrument médical selon l'une des revendications précédentes, **caractérisé en ce que** le pourtour de l'arbre d'entraînement (22) coopérant avec le dispositif contact est recouvert d'un matériau résistant et bon conducteur électrique, de préférence de l'or.

11. Instrument médical, qui comprend à son extrémité distale (14) un instrument (16) alimenté en énergie à haute fréquence (HF), ainsi qu'un raccordement électrique HF (24) et une amenée de courant HF (21), qui s'étend d'une extrémité proximale (18) de l'instrument jusqu'à son extrémité distale (14), sachant que l'amenée de courant HF (21) est conçue comme un arbre d'entraînement (22) pour un mécanisme d'entraînement rotatif de l'instrument (16) et un dispositif de contact (40') est prévu qui est relié électriquement au raccordement électrique HF (24) et vient au contact de l'arbre d'entraînement (22) de manière électrique, **caractérisé en ce que** le dispositif de contact (40') comprend un boîtier (71), qui reçoit une section longitudinale de l'arbre d'entraînement (22) et dans lequel un fluide conducteur électrique est contenu, de telle sorte qu'une connexion électrique se produit entre l'arbre d'entraînement (22), le fluide et par conséquent le raccordement électrique HF (24).
